# EUROPEAN PATENT APPLICATION

(11) **EP 0 869 187 A2**
(43) Date of publication of application: **07.10.1998**
(21) Application number: 98102695.8
(22) Date of filing: 17.02.1998
(51) Int. Cl.: C12Q 1/68

(54) **Replication of nucleic acids using single-strand DNA binding proteins**

(30) Priority: 24.02.1997 US 805100
(71) Applicant: Becton, Dickinson and Company, Franklin Lakes, New Jersey 07417-1880 (US)
(72) Inventor: Fraiser, Melinda S., Durham, North Carolina 27704 (US)
(74) Representative: Rambelli, Paolo

(57) **Abstract**

Single-strand DNA binding proteins have been found to increase the efficiency of strand-displacement replication of long targets, and when added to amplification reactions such as SDA, they significantly improve the amplification efficiency of targets which are otherwise too long to be efficiently amplified. The gp32 protein of T4 has been shown in SDA to produce about 10⁶ to 10⁸-fold amplification in 15-20 min. for targets about 800-1,000 bp in length. In the absence of gp32 there is essentially no detectable amplification for targets of this length. Targets about 400-500 bp in length are amplifiable about a billion-fold in the presence of gp32, representing an amplification factor approximately equivalent to that observed for targets less than 100 bp in length in conventional SDA reactions in the absence of SSBs. Other single-strand DNA binding proteins have also been shown to improve replication and amplification efficiency for long targets.

SEQUENCE LISTING

(1) GENERAL INFORMATION:

(i) APPLICANT: BECTON, DICKINSON AND COMPANY
(A) NAME: BECTON, DICKINSON AND COMPANY
(B) STREET: 1 Becton Drive
(C) CITY: Franklin Lakes
(D) STATE: NJ
(E) COUNTRY: US
(F) ZIP: 07417

(ii) TITLE OF INVENTION: Replication of Nucleic Acids Using
Single-Strand DNA Binding Proteins

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(v) CURRENT APPLICATION DATA:
(A) APPLICATION NUMBER:
(B) FILING DATE:

(2) INFORMATION FOR SEQ ID NO:1:

(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:1:

ACCGCATCGA ATGCATGTCT CGGGTAAGGC GTACTCGACC 40

(2) INFORMATION FOR SEQ ID NO:2:

(i) SEQUENCE CHARACTERISTICS:
(A) LENGTH: 13 base pairs
(B) TYPE: nucleic acid
(C) STRANDEDNESS: single
(D) TOPOLOGY: linear

(xi) SEQUENCE DESCRIPTION: SEQ ID NO:2:

CGCTGAACCG GAT 13

## Description

### FIELD OF THE INVENTION

The invention relates to methods for amplification of nucleic acid target sequences, and in particular to the use single-strand DNA binding proteins in nucleic acid amplification reactions.

### BACKGROUND OF THE INVENTION

*In vitro* nucleic acid amplification techniques are powerful tools for detection and analysis of small amounts of nucleic acids, and the high degree of sensitivity of these methods has generated interest in developing them for diagnosis of infectious and genetic diseases, isolation of genes for analysis, and detection of specific nucleic acids as in forensic medicine. Nucleic acid amplification methods include, for example, the Polymerase Chain Reaction (PCR), the Ligase Chain Reaction (LCR), Self Sustained Sequence Replication (3SR), Nucleic Acid Sequence Based Amplification (NASBA), Transcription Mediated Replication (TMR) and Strand Displacement Amplification (SDA). Strand Displacement Amplification is an isothermal amplification reaction which is capable of producing greater than a billion-fold amplification of a target sequence in less than 30 min. at constant temperature (G. T. Walker, et al. 1992. *Proc. Nat. Acad. Sci. USA* **89**, 392-396; G. T. Walker, et al. 1992. *Nuc. Acids. Res.* **20**, 1691-1696; U.S. Patent No. 5,455,166; U.S. Patent No. 5,270,184; EP 0 684 315).

The SDA reaction may be conducted at a constant temperature between about 35°C and 45°C or at constant higher temperatures to improve amplification efficiency and specificity (referred to as thermophilic SDA or tSDA and described in published European Patent Application No. 0 684 315). In either format, SDA employs 1) a restriction endonuclease which nicks a hemimodified restriction endonuclease recognition/cleavage site and 2) a polymerase which extends from the nick and displaces a copy of the target sequence while polymerizing a new strand using the target sequence as a template. Repeated cycles of nicking and displacing produce additional copies of the target sequence. SDA is unique among nucleic acid amplification methods in its use of the strand displacing ability of the DNA polymerase to separate complementary strands for subsequent cycles of target replication. This process is referred to as strand displacement replication.

The SDA reaction is described in U.S. Patent No. 5,455,166, U.S. Patent No. 5,270,184 and EP 0 684 315. The steps of the SDA reaction are the same regardless of the temperature and enzymes employed, and it requires several specific enzymatic activities in order to amplify a target sequence. The SDA polymerase must 1) lack 5'-3' exonuclease activity, either naturally or by inactivation, 2) incorporate the derivatized deoxynucleoside triphosphates (dNTPs) required by SDA (nucleotide analogs such as αthio-dNTPs), and 3) displace a downsteam single strand from a double stranded molecule starting at a single stranded nick. The SDA restriction endonuclease must 1) nick (i.e., cleave a single strand of) its double stranded recognition/cleavage site when the recognition/cleavage site is hemimodified, and 2) dissociate from its recognition/cleavage site to allow the polymerase to bind and amplify the target. Incorporation of the dNTP analog into the restriction endonuclease recognition site induces nicking by the restriction endonuclease. Thiolated dNTPs are the most commonly used nucleotide analog in SDA, however, incorporation of methyl- of boron-substituted dNTPs also induces nicking. Examples of polymerases and restriction endonucleases having the appropriate biological activities for SDA are described in the patent publications cited above. Terms relating to amplification by SDA are defined in EP 0 684 315.

SDA is a fast and highly efficient amplification process, but suffers from limitations in the length of the target which can be efficiently amplified. That is, it has been observed that SDA is most efficient when amplifying short targets (typically about 100 bp or less), and that amplification factors decrease between 5- and 100- fold for each 50 bp increase in target length. Decreases in amplification factors from 4.5 X 10⁸ (52 bp target) to 3.0 X 10⁴ (198 bp target) have been observed. This is unlikely to be due to reduced efficiency of nicking by the restriction endonuclease (which would be expected to be independent of target length), but may be the result of the DNA polymerase terminating amplification products before polymerization and displacement are complete. Premature termination of replication would result in an aborted, partial amplicon which cannot be further amplified, and the potential for such premature terminations may increase as the length of the target increases, thus reducing the efficiency of the amplification reaction. Many factors could contribute to the inability of SDA to efficiently amplify longer targets, including local sequences within the target which attenuate the reaction, low polymerase processivity, inefficient displacement of longer strands, switching of the polymerase to replication of the displaced strand, negative effects of the modified dNTPs or dUTP, etc. In contrast, the PCR does not require strand displacement replication by the DNA polymerase. In the PCR total separation of the complementary strands is accomplished by applying high temperatures, and the PCR is capable of efficiently amplifying targets several hundreds of base pairs in length.

The presence of organic solvents has been shown to improve the efficiency of SDA and increase the amount of amplification product produced, but they do not increase the length of the target which can be detectably amplified. Solvents do not have a specific effect on the performance of the DNA polymerase, but would be expected to affect many aspects of the reaction, including primer hybridization, strand displacement efficiency, and general enzyme activity. There is therefore a need to discover reagents and/or methods which improve the amplification efficiency of SDA for long targets, particularly targets greater than about 100 bp in length.

Single-strand DNA binding proteins (SSBs) have a high, sequence non-specific affinity for single-stranded DNA (ssDNA) and a comparatively low affinity for double-stranded DNA. Most also have a strong affinity for single-stranded RNA (ssRNA). They are usually required for DNA replication, recombination and repair of the organism's genome. SSBs specifically stimulate their cognate DNA polymerase, enhance the fidelity of DNA synthesis, improve the processivity of the DNA polymerase by destabilization of the helix, promote polymerase binding and organize and stabilize the replication origin. Many SSBs also participate in recombination and facilitate renaturation of DNA. SSBs have been identified and characterized in many species (J. W. Chase and K. R. Williams. 1986. *Ann. Rev. Biochem.* 55:103-136), including bacteriophage T4 (gp32 protein; K. R. Williams, et al. 1980. *Proc. Natl. Acad. Sci*. 77:4614-4617; D. P. Giedroc, et al. 1986. *Proc. Natl. Acad. Sci*. 83:8452-8456; D. K. Sandhu and P. Keohavong. 1994. *Gene* 144:53-58; D. P. Giedroc, et al. 1990. *J. Biol. Chem.* 265:11444-11455), bacteriophage T7 (gene 2.5 protein; Y. T. Kim, et al. 1992. *J*. *Biol. Chem.* 267:15022-15031), bacteriophage Φ29 (protein p5; C. Gutierrez, et al. 1991. *J*. *Biol. Chem*. 266:2104-2111), yeast (S. G. LaBonne and L. B. Dumas. 1983. *Biochem*. 22:3214-3219; A. Y. S. Jong, et al. 1985. *J. Biol. Chem*. 260:16367-16374; E. Alani, et al. 1992. *J. Mol. Biol*. 227:54-71; E. Van Dyck, et al. 1992. *EMBO* 11:3421-3430) and adenovirus (G. Kitchingman. 1995. *Virology* 212:91-101; P. N. Kanellopoulos and H. Van Der Zandt. 1995. *J. Struct. Biol*. 115:113-116).

SSBs have been used *in vitro* to enhance hybridization of probes to a nucleic acid target (U.S. Patent Nos. 5,534,407 and 5,449,603) and to promote specific alignment of adjacent probes hybridized to a nucleic acid polymer (U.S. Patent No. 5,547,843). SSBs have also been used as labeling means for detection of ssDNA (U.S. Patent Nos. 4,963,658 and 5,424,188). T4 gp32 has been used to enhance polymerase activity for cloning by PCR (U.S. Patent No. 5,354,670) and the gp32 gene has been used in expression systems for production of recombinant proteins (U.S. Patent No. 5,182,196). The Φ29 SSB has been shown to stimulate replication of Φ29 DNA by increasing the number of reinitiations on new templates (G. Martin, et al. 1989. *Nucl. Acids Res.* 17:3663-3672; C. Gutierrez, et al. 1991. *J. Biol*. *Chem.* 266:2104-2111). However, it has not previously been recognized that the length of the target which can be efficiently replicated by strand displacement replication *in vitro* or amplified in a nucleic acid amplification reaction requiring strand displacement replication is increased in the presence of SSBs.

### SUMMARY OF THE INVENTION

It has now been found that single-strand DNA binding proteins (SSBs), when added to amplification reactions such as SDA, significantly improve the amplification efficiency of targets which are otherwise too long to be efficiently amplified. As the SSBs appear to be affecting the strand displacement replication step of the reaction to increase the length of the template which can be replicated before dissociation of the polymerase, it is believed that SSBs will similarly improve the replication efficiency of long targets in other reactions which involve strand displacement replication steps. Of the SSBs tested in the course of making the invention, gp32, yeast RPA-1 and Φ29 protein 5 are the most effective for increasing the efficiency of long target replication in strand displacement replication reactions. This results in significantly improved amplification efficiencies in amplification reactions which employ strand displacement replication and allows efficient amplification of targets the length of which precludes detectable amplification in the absence of SSBs. For example, gp32 has been shown to result in about 10⁶ to 10⁸-fold amplification in 15-20 min. for targets about 800-1,000 bp in length. In contrast, there is essentially no detectable amplification for targets of this length in the absence of SSB. Targets about 400-500 bp in length may be amplified about a billion-fold in the presence of gp32, representing an amplification factor approximately equivalent to that observed for targets less than 100 bp in length in conventional SDA reactions which do not contain SSBs.

It is an unexpected discovery that the SSBs function to improve strand displacement replication in heterologous enzymatic replication and amplification systems such as SDA. Many studies on the functions and activities of SSBs have shown that the SSB and the polymerase with which it is paired must be homologous (i.e., from the same species) in order for SSB function to be efficiently expressed. In contrast, in SDA the polymerase is typically from a species of organism which is different from that of the SSB (e.g., a *Bacillus* polymerase used with a bacteriophage SSB). Under the reaction conditions of SDA these heterologous polymerase/SSB pairs were surprisingly highly efficient at increasing the amplifiable target length.

### DESCRIPTION OF THE DRAWINGS

Fig. 1 illustrates the effect of SSBs on amplification efficiency in linear SDA screening assays at 40°C and 50°C.

Fig. 2 illustrates the effect of gp32 on amplification efficiency of targets of increasing length.

### DETAILED DESCRIPTION OF THE INVENTION

It was hypothesized that the enzyme most likely to be negatively affected by the length of the target in reactions requiring strand displacement replication was the polymerase. Methods for enhancing the action of the polymerase were therefore explored in an effort to facilitate amplification of long targets. A target or target sequence is a selected oligonucleotide to be replicated or amplified and, optionally, detected. As used herein, "long targets" are defined as oligonucleotide sequences which are too long to be efficiently replicated or amplified in conventional reactions requiring strand displacement replication. Long targets are typically 100 nucleotides or more in length. For example, a long target for a nucleic acid amplification reaction or a strand displacement replication reaction is generally between about 100 and about 5,000 nucleotides long, preferably between about 150 and about 2,000 nucleotides long. For targets at the upper end of these size ranges (e.g., between about 2,000 and about 5,000 nucleotides long), it may be useful to increase the reaction time to obtain detectable amplification or replication products. The appropriate reaction time for a long target of a given length may be determined empirically using routine methods known in the art.

SSBs are replication accessory proteins and were examined for their effect on strand displacement synthesis such as occurs in SDA. For testing, a simple, non-exponential system was devised. This was essentially a linear SDA reaction without primers which nonetheless employed the basic nicking and extension reactions central to SDA. A double-stranded DNA template with a nickable restriction site near the 5' end and full substitution with a thiolated dNTP was synthesized. When this template was incubated with the appropriate restriction enzyme, polymerase, deoxynucleoside triphosphates and buffer to simulate SDA-like reaction conditions, the hemi-thiolated restriction site was nicked and the polymerase extended from the nick to produce a single-stranded product by strand displacement synthesis. As in SDA, strand displacement synthesis reproduced the nickable restriction site, allowing the process to repeat. The effect of added reagents on the nicking and strand displacement process could be observed in this test system as a model for SDA and other reactions comprising strand displacement replication steps.

To set up the linear SDA screening system, a pUC plasmid was modified by inserting SDA priming regions on either side of the multiple cloning site. Then, various lengths of lambda DNA were inserted into the BamHI site of the vector. The resulting "SDA target plasmid" provided a range of insert lengths which could all be amplified using a single pair of amplification primers. For SDA, a target must contain a hemi-thiolated restriction site (e.g., BsoBI) and have a defined 3' end. To provide such targets, the SDA vectors with the various lambda inserts were PCR amplified for 30 cycles. The primers used for the PCR were an SDA amplification (S) primer (ACCGCATCGAATGCATGTCTCGGGTAAGGCGTACTCGACC, SEQ ID NO:1) and an SDA bumper primer (CGCTGAACCGGAT, SEQ ID NO:2). dCTPαS was fully substituted for dCTP in the reaction to create the hemi-modified BsoBI site. The PCR reaction consisted of 1X Promega thermo DNA polymerase buffer (50 mM KCl, 10 mM Tris-HCl, pH 9.0 at 25°C, and 1.0% Triton X-100), 3.9 mM MgCl₂ (1 mM free MgCl₂), 0.1 µ M each primer, 1.4 mM dCTPαS, 0.5 mM each dGTP, dATP and TTP, 1 ng SDA plasmid, 5 µL Sequencing grade Taq polymerase (Promega). To amplify the vector, the reaction mix was overlaid with mineral oil and heated to 95°C for 5 minutes. The polymerase was added at 95°C for a hot start and thirty, one minute temperatures cycles at 96°C, 55°C and 72°C were performed. Each PCR product for linear SDA was purified by passage over a Centricon 100 column followed by proteinase K digestion (1.25 µg) for 30 minutes at 65°C. The SDA templates were chloroform extracted, ethanol precipitated and quantitated by spectroscopy.

Numerous examples of SSBs have been isolated from a wide variety of sources, from bacteriophage to eukaryotes. The following SSBs were tested in the linear SDA system to evaluate their effect on the efficiency of amplification of long targets: replication protein A-1 (rpa-1) and replication in mitochondria protein (rim-1) from *Saccharomyces cerevisiae*, gene 2.5 protein from T7 (gp2.5), protein p5 of bacteriophage Φ29 (p5), gene 32 protein of T4 (gp32) and *E. coli* SSB. The SDA reaction was initally developed using mesophilic polymerases and restriction endonucleases and was conducted at about 35-45°C. This produced a highly efficient amplification system. The later improvement of tSDA, using thermostable polymerases and restriction endonucleases, resulted in reduced background, increased amplification efficiency and a significant reduction in the time required for the reaction. The temperatures of mesophilic SDA are compatible with the SSBs, which so far have been derived from mesophilic organisms with temperature optima for growth between about 25°C and 37°C. However, as the most efficient SDA amplification system is the thermophilic reaction which operates at about 45-60°C (i.e., tSDA), it would be advantageous to use the SSBs at tSDA temperatures to further improve and enhance amplification. The known mesophilic SSBs were not expected to be stable at the temperatures of tSDA.

The linear SDA assay was performed to test the efficacy of the SSBs at both 40°C and 50°C. Varying amounts of the SSBs were tested in reactions containing 25 mM KᵢPO₄ pH 7.5, 0.1 µg/µL BSA, 1.4 mM dCTPαS, 0.5 mM each dATP, dGTP and TTP (total 2.9 mM dNTPs), 6.9 mM MgCl₂ (4 mM free MgCl₂), 0.5 µL each α³²P-TTP and dATP (3,000 Ci/mm) and 10 nM 400-mer SDA template prepared as described above. All reaction components except the enzymes and SSBs were assembled and prewarmed at the reaction temperature for 5 minutes. Alter prewarming, 25 units BsoBI and 50 units exo⁻ Klenow (40°C reactions) or 8 units Bca polymerase (50°C reactions) were added with varying amounts of the SSB. The reactions were incubated at 40°C for 30 minutes or 50°C for 15 min. The control reaction to measure the amount of amplification product generated during a single cycle of linear SDA contained the same buffer components, the same amount of SDA template and the same polymerase as the SSB reactions, but did not include restriction enzyme or SSB. The control reaction also contained 166 µM of the bumper primer used to generate the SDA templates (see above). The reactions were terminated by addition of an equal volume of 95% formamide, 20 mM EDTA, 0.05% each bromphenol blue/xylene cyanol and treatment with 0.5 µg of proteinase K for 30 minutes at 65°C. The samples were denatured for 3 minutes at 100°C and quick chilled on ice prior to electrophoresis on a 6% sequencing gel. The gel was analyzed on the PhosphorImager (Molecular Dynamics) to obtain pixel values indicating the amount of 400-mer displaced, linear SDA product generated. The PhosphorImager provides quantitation by attributing a relative pixel value to the amount of radioactivity present in the selected band on the gel and subtracting the measured background of each lane from this value. The lane containing the control reaction was used to quantitate the amount of product obtained from a single extension event. The number of displaced strands (SDA turnover events) generated in the test reactions was determined by dividing the amount of 400-mer amplification product in the SSB reaction lanes by the amount of product obtained from a single extension event. Dividing the total number of displaced strands by the duration of the reaction provided the number of SDA cycles per minute in the reaction (i.e., the rate of amplification).

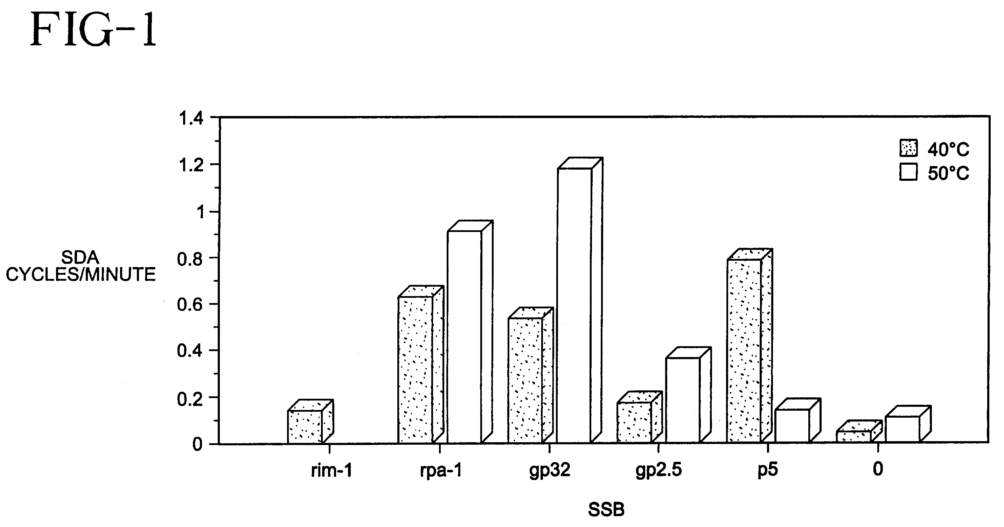

Yeast rpa-1, gp2.5, p5 and gp32 were tested at both reaction temperatures. Rim-1 and *E. coli* SSB were tested at 40°C only. The results are shown in Fig. 1. With the exception of the *E. coli* SSB, each of the SSBs tested improved generation of the 400 base linear SDA product at 40°C. The greatest amount of amplification product at this temperature was generated using 0.24 µg/µL p5 (0.79 cycles/min.). In addition, gp32 (0.196 µg/µL) and rpa-1 (0.024 µg/µL) performed well (0.54 and 0.64 cycles/min., respectively). Even the least effective SSBs under these conditions (gp2.5, 0.18 cycles/min. and rim-1, 0.15 cycles/min.) produced at least a 3 fold improvement over the amount of product generated in the absence of SSB (0.05 cycles/min.). Unexpectedly, at 50°C there was also a significant improvement in amplification efficiency in the presence of all but one of the SSBs tested. The gp2.5 protein produced a 3-fold improvement in amplification efficiency (0.37 cycles/min.), gp32 produced a 10-fold improvement (1.2 cycles/min.) and rpa-1 produced about an 8-fold improvement (0.92 cycles/min.) as compared to reactions without SSBs (0.12 cycles/min.). The magnitude of the improvement for p5 (0.14 cycles/min.) was minimal but still detectable over the efficiency of the reaction without SSB. Enhancement of long target amplification efficiency at 50°C was not predicted based on the temperature ranges for growth of the source organisms.

The results and analyses of linear SDA as an assay system indicate that strand displacement replication is the step which limits the length of the target which can be efficiently amplified or replicated. The linear SDA screening assay described here may therefore be used routinely to screen additional known SSBs and new SSBs as they are discovered to evaluate their utility for improving the efficiency of long target strand displacement replication, particularly in nucleic acid amplification reactions which employ strand displacement replication mechanisms. By selecting the length of the long target sequence to be linearly amplified in the screening assay, it is also possible to evaluate the relative ability of the SSB to promote efficient strand displacement replication of long targets. Further, the results of the linear amplification screening assays are predictive of improved long target amplification efficiency in exponential amplification reactions employing strand displacement replication, as the same reaction mechanisms are present in both the linear and exponential reactions. Of the six SSBs screened here, all but one demonstrated the activities and utilities necessary for the practice of the invention. Four of the five active SSBs were highly effective for improving the amplification efficiency of long targets and one wasmoderately effective. Therefore, using the screening methods disclosed herein, the practitioner may routinely identity additional SSBs having the described properties and utilities.

The use of SSBs in SDA reactions to increase amplifiable target length is compatible with various detection systems known for detection of nucleic acid amplification. For example, hybridization of labeled probes or hybridization and extension of a detector probe as described by Walker, et al. (*Nucl. Acids Res., supra*) are useful means for detecting the amplification products of amplification or replication in the presence of SSBs. A preferred method for detecting the amplification products of SDA is the signal primer system described in U.S. Patent No. 5,547,861 and U.S. Patent No. 5,550,025, and these methods are also useful in SDA in the presence of SSBs.

### EXAMPLE 1

Gp32 was selected for further study in exponential tSDA amplification systems. In a first experiment, 10⁸-fold amplification of a 500 bp target was demonstrated with addition of 10 µg of gp32. In a second experiment to assess the detection sensitivity of the amplification reaction, varying amounts of the 500 bp target were added in a volume of 5 µL to 40 µL of reaction buffer (25 mM KᵢPO₄ pH 7.6, 0.1 mg/mL BSA, 0.5 µM S1 and S2 SDA amplification primers, 0.05 µM B1 and B2 SDA bumper primers, 1.4 mM dCTPαS, 0.5 mM dUTP, 0.2 mM dATP, 0.2 mM dGTP, 10.5% glycerol, 7 mM magnesium acetate, 500 ng human placental DNA). The SDA amplification primers and bumper primers used in this experiment are described by C. A. Spargo, et al. (1996. *Molec. Cell. Probes* **10**, 247-256). Human placental DNA only (100 ng/µL) was added to zero-target control samples. The samples were denatured for 3 minutes at 100°C in a boiling water bath, transferred to 52°C and equilibrated for 4 minutes. The enzymes (30 units AvaI, 36 units exo⁻ Bst) and SSB (10 µg gp32) were added to each sample in a volume of 5 µL, mixed and placed at 52°C for 30 minutes. The reactions were terminated by heating at 100°C for 5 minutes. Amplification products were detected by hybridization and extension of an end-labeled detector probe as described by Walker, et al. (*Nucl. Acids Res., supra*). Six µL of a detection cocktail (5 µL of 35 mM KᵢPO₄ pH 7.6, 6 mM MgOAc, 1 mM each dNTP and 1 µL of labeled probe) was added to the SDA reaction. After denaturing at 100°C for 3 minutes, the reactions were transferred to 37°C and equilibrated for 3 minutes. Two µL of exo⁻ Klenow (1 U/µL in 50% glycerol) were added and the extension reaction was incubated at 37°C for 15 minutes. The reaction was terminated by addition of 13 µL of formamide stop solution (90% formamide, 0.05% BPB, 1X TBE). Half of each sample (13 µL) was loaded on an 8% sequencing gel and electrophoresed at 57 Watts for about 1 hour. dUTP was included in this experiment to allow amplicon decontamination using UDG, however, TTP may be substituted for dUTP, in which case it is advantageous to increase the phosphate buffer to 30 mM and include TTP in the same amount as the other dNTPs (typically about 0.2 mM).

Regardless of its initial concentration, no detectable amplification of the 500 bp target was seen in the absence of SSB. In contrast, target amplification was readily detectable when gp32 was present, indicating a significant improvement in amplification efficiency due to the SSB. The amplification factors in the presence of gp32 and dUTP are shown in the following Table:

**TABLE**

| # INITIAL TARGETS | APPROXIMATE AMPLIFICATION FACTOR |
|---|---|
| 10³ | 5.6 X 10⁷ |
| 10² | 1.5 X 10⁸ |
| 10 | 5 X 10⁷ |

In the presence of TTP instead of dUTP, amplification factors increased about 2-fold. As few as 10 molecules of the 500 bp target were readily detectable in these experiments, demonstrating a very sensitive assay for a long target. The experiments were repeated in a similar protocol, with similar results, using BsoBI and exo⁻ Bst polymerase. These reactions achieved amplification factors of about 10⁷ to 10⁸ for the 500 bp target in both the TTP and dUTP systems. These amplification factors are only slightly less than the amplification factors typical of conventional SDA of targets less than 100 bp in length (i.e., short targets with no added SSB).

### EXAMPLE 2

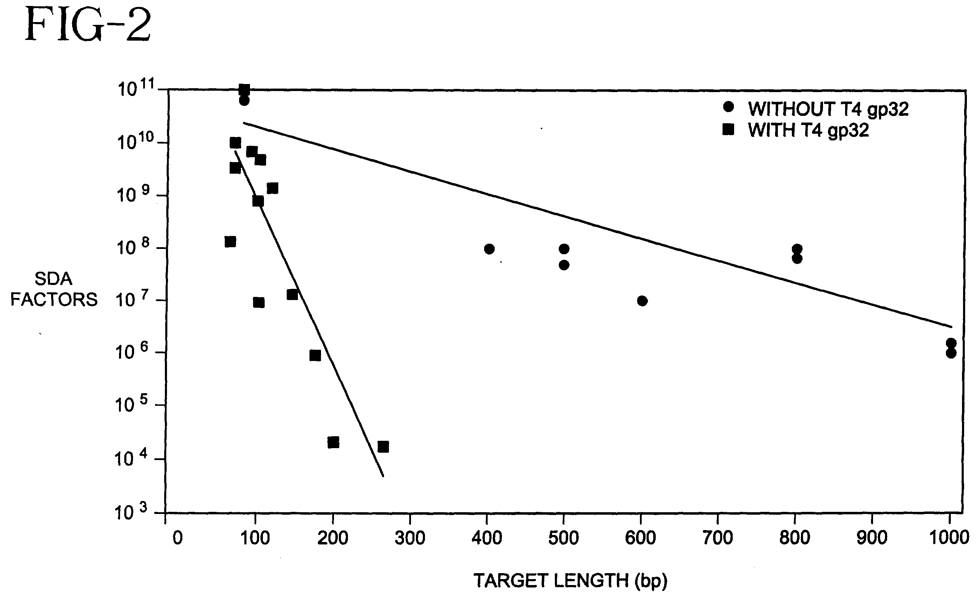

To explore the effect of varying target length on amplification in the presence of SSBs, targets of varying lengths were selected from the lambda SDA templates and tested for amplification in the presence of gp32. As shown in Fig. 2, in the absence of gp32 amplification factors drop precipitously as target length increases, from about 10¹⁰ for the 100 bp target to about 10⁴ for the 200 bp target. Amplification is essentially undetectable as the target length approaches about 300 bp. In contrast, gp32 substantially prevents any reduction in amplification efficiency between 100 and 200 bp, and allows targets about 1,000 bp in length to be amplified about 10⁶-fold. In practice, addition of SSB to the amplification reaction typically produces about 10⁸-fold amplification with 10-100 molecule detection sensitivity for a 500 bp target, about 10⁸-fold amplification with 10-100 molecule detection sensitivity for an 800 bp target (using TTP), and about 10⁶-fold amplification with 100-1000 molecule detection sensitivity for a 1,000 bp target (using TTP). Based on these results, SSBs should produce improvements in amplification and strand displacement replication efficiency for targets at least about 100 nucleotides long, e.g., between about 100 and about 5,000 nucleotides long. In most replication and amplification reactions, however, the preferred long target is generally between about 150 and about 2,000 nucleotides in length. These long targets are readily replicated or amplified using SSBs to enhance amplification efficiency, although increasing target length may necessitate increasing reactions times in order to detect the reaction product.

SSBs are useful for improving long target amplification efficiency for both DNA and RNA targets (reverse transcription amplification) with significant increases in the length of the target which may be efficiently amplified. SSB enhancement of long target amplification efficiency may also be applied to *in situ* amplification and replication reactions, for example as described in U.S. Patent No. 5,523,204. While not wishing to be bound by any particular mechanism by which the invention operates, Applicants hypothesize that the SSBs bind to the displaced single strand, facilitating movement of the replication fork and inhibiting branch migration between the strand being extended and the strand being displaced when the polymerase dissociates during non-processive replication. Such a mechanism would explain the marked reduction in the number of replication aborts observed during SDA, which may also contribute to efficient amplification of long targets. The observed decrease in non-specific background amplification may also be attributed to this mechanism, as the same SSB activities might also result in a reduction in mispriming. These experiments demonstrate improved efficiency of strand displacement replication of long targets in the presence of SSBs. The strand displacement replication reaction is central to SDA, and enhancement of long target amplification efficiency has been illustrated in SDA as a model for strand displacement replication reactions. However, similar increases in the length of the target which can be replicated would be expected in any reaction which employs a DNA polymerase to perform strand displacement replication.

## Claims

1. A method for increasing the efficiency of replication of a long target comprising replicating the long target in a strand displacement replication reaction, the reaction comprising a strand-displacing DNA polymerase and a single-strand DNA binding protein, wherein the single-strand DNA binding protein is present in an amount sufficient to increase the efficiency of replication of the long target as compared to an efficiency of replication of the long target in the absence of the single-strand DNA binding protein.

2. The method of Claim 1 wherein the strand displacement replication reaction occurs in a nucleic acid amplification reaction.

3. The method of Claim 2 wherein the nucleic acid amplification reaction is Strand Displacement Amplification (SDA).

4. The method of Claim 1 wherein the single-strand DNA binding protein is selected from the group consisting of gp32, rpa-1, rim-1, gp2.5 and p5.

5. The method of Claim 1 wherein the long target is between about 100 and about 5,000 nucleotides in length.

6. A method for increasing the efficiency of amplification of a long target comprising amplifying the long target in an amplification reaction comprising a strand displacement replication step, the amplification reaction comprising a strand-displacing DNA polymerase and a single-strand DNAbinding protein, wherein the single-strand DNA binding protein is present in an amount sufficient to increase the efficiency of amplification of the long target as compared to an efficiency of amplification of the long target in the absence of the single-strand DNA binding protein.

7. The method of Claim 5 wherein the amplification reaction is Strand Displacement Amplification.

8. The method of Claim 5 wherein the single-strand DNA binding protein is selected from the group consisting of gp32, rpa-1, rim-1, gp2.5 and p5.

9. The method of Claim 6 wherein the long target is between about 100 and about 5,000 nucleotides in length.

10. The method of Claim 9 wherein the long target is between about 150 and about 2,000 nucleotides in length.
